Europäisches Patentamt

European Patent Office

Office européen des brevets

(19)

(11) Publication number: **0 180 138**
A2

# (12) EUROPEAN PATENT APPLICATION

(21) Application number: 85113449.4

(22) Date of filing: 23.10.85

(51) Int. Cl.⁴: **G 01 N 27/46**, G 01 R 29/24

(30) Priority: 02.11.84 US 667935

(43) Date of publication of application: 07.05.86
Bulletin 86/19

(84) Designated Contracting States: DE FR GB IT

(71) Applicant: GENERAL ELECTRIC COMPANY, 1 River
Road, Schenectady New York 12305 (US)

(72) Inventor: Niedrach, Leonard William, 1403 Myron Street,
Schenectady New York 12309 (US)
Inventor: Will, Fritz Gustav, 102 Berkley Square, Scotia
New York 12302 (US)

(74) Representative: Schüler, Horst, Dr. European Patent
Attorney et al, Kaiserstrasse 41,
D-6000 Frankfurt/Main 1 (DE)

(54) Oxygen sensor with residual life indicator.

(57) A residual life indicator for a portable long life oxygen
sensor. A circuit of the sensor includes a sensor electrode and
counter electrode connected through a resistor network and a
voltmeter calibrated to oxygen partial pressure. An electrolyte
also connects the electrodes. A coulometer and temperature
compensated resistor network is included in the sensor circuit-
ry and provides visual indication of life expectancy.

OXYGEN SENSOR WITH RESIDUAL LIFE INDICATOR

BACKGROUND OF THE INVENTION

The present invention relates to oxygen sensors suitable for use in spacecraft and for other uses. More specifically, it relates to determining the remaining overall or total life of oxygen sensors such as are used on the space shuttle.

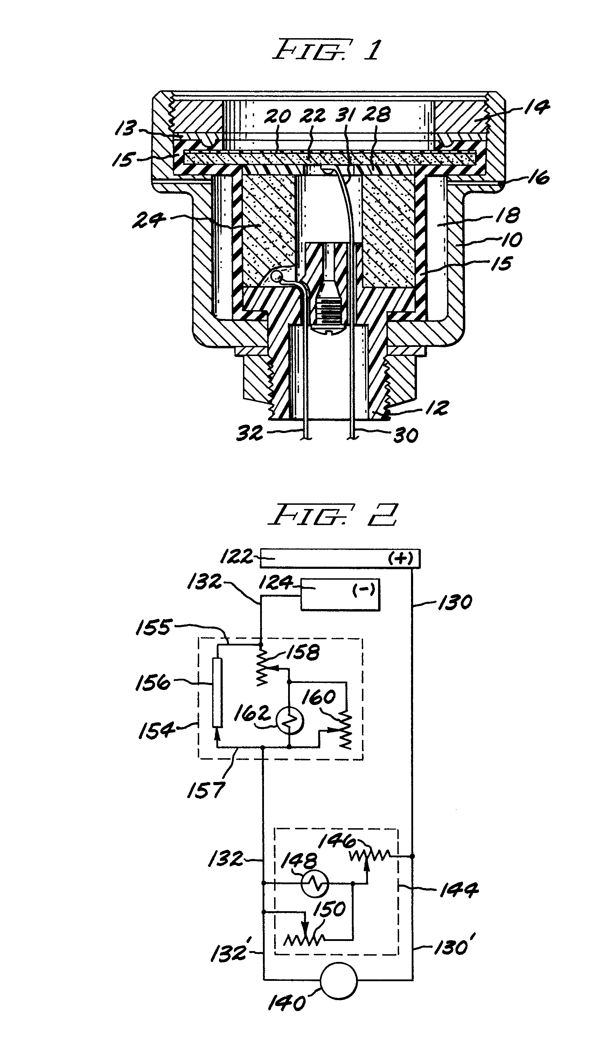

It has been observed that an oxygen sensor as illustrated in Figure 1 has a total life of approximately 8000 hours when exposed to 1 atmosphere of air at 75°F. Total life includes both useful operating life and exposure to oxygen for testing, storage and for any other reason. The calculated total life of a sensor as illustrated in Figure 1 is approximately 9200 hours. The original specifications of the National Aeronautics and Space Administration for such sensors is 6236 operational hours of useful operating life.

An application of the same inventors, Serial No. 641,650, filed August 17, 1984, and assigned to the same assignee as the subject application, teaches means and methods by which the life of oxygen sensors may be extended.

Because substantial testing and operation of sensors is necessary prior to an actual mission, an appreciable portion of the total life of such a sensor is used up in laboratory testing, in the break-in period, in various calibrations and in an air exposure aboard the spacecraft before the scheduled launch. This can be further extended by delays in the actual launch thus using up a further portion of the total life of the sensor. In all, a significant fraction of the total life of a sensor may be used in this way.  For these and other reasons, reduced sensor output has mandated premature sensor replacement prior to a number of space shuttle missions.  There is considerable interest in NASA in oxygen sensors having much longer operating life for space applications and in knowing what remaining operating life a sensor has.

State of the art oxygen sensors have no means of residual life indication.  The life of a sensor depends in part on temperature and on oxygen partial pressure.  It also depends on and is affected by laboratory tests and pre-launch air exposure.  Residual or remaining operating life cannot be estimated satisfactorily.

The lack of ability to estimate residual operating life satisfactorily has led to the need for occasional last-minute replacement of sensors prior to launch of a spacecraft and sometimes to premature failure of sensors during a mission.

BRIEF STATEMENT OF THE INVENTION

Accordingly, it is one object of the present invention to improve the design of oxygen sensors for space and other applications so that expected life can be determined.

Another object is to provide an oxygen sensor having a useful life which is determinable from inspection of the device.

Another object is to provide an oxygen sensor for space applications which has an expected life which is readily determinable.

A further object is to provide a design of an oxygen sensor which has a residual life indicator built therein.

-2-

Other objects and advantages of the present invention will be, in part, apparent and, in part, pointed out in the description which follows.

In one of its broader aspects, objects of the present invention may be achieved by providing a portable oxygen sensor having a residual life indicator. The portable oxygen sensor includes a sensing electrode adapted to reduce oxygen which permeates thereto through a diffusion barrier, a counter electrode of porous copper metal which determines the life of the sensor, a set of conductors, a resistor network and a coulometer network connected in series and providing an electrical connection between the electrodes. An aqueous alkaline solution provides an electrolytic path between the sensing electrode and the counter electrode. A coulometer suitably incorporated into the coulometer network provides a visual indication of residual life of the counter electrode.

## BRIEF DESCRIPTION OF THE DRAWINGS

The description which follows will be better understood by reference to the accompanying drawings in which:

FIGURE 1 is an axial sectional view of an oxygen sensor as provided by the prior art.

FIGURE 2 is a schematic drawing of a sensing and display circuit as provided in connection with the present invention.

FIGURE 3 is a top plan view of the external casing of a sense and display mechanism as provided pursuant to this invention.

FIGURE 4 is a side elevational view in part in section of the sense and display mechanism of Figure 3.

-3-

## DETAILED DESCRIPTION OF THE INVENTION

The sensor of Figure 1 is essentially a copper-air battery. It includes an outer casing 10, which is preferably a polymeric material, having an insulating bushing 12 at its lower end and an externally threaded plastic insert 14 at its upper end. Between the bushing 12 and the insert 14 are essential elements of the sensor. An impervious washer 13 is positioned between the upper portion of bladder 15 and insert 14. This holds the top inwardly extending portion of bladder 15 against the outer portions of membrane 20 and defines the area of membrane 20 exposed to the atmosphere. An expansion bladder 15, of neoprene or other suitable rubber such as ethylene propylene rubber, laterally surrounds the essential components of the battery and is positioned between these elements and the casing 10. The bladder 15 is spaced from the walls of the casing in the lower portion of the casing to permit its lateral expansion and vent holes 16 provide means by which gas pressure is equilibrated in the space 18, between the casing and the bladder, and the outside atmosphere. At the upper part of the essential elements of the sensor is a polymer membrane 20. The polymer membrane has the capacity to perfuse and pass oxygen at a rate which is proportional to the partial pressure of oxygen in contact with the exterior surface of the membrane. Oxygen which is in contact with the exterior of the sensor passes through the membrane 20 and into contact with a sensing electrode 22.

The sensing electrode is porous metal and it is gold plated. The function of the gold on the sensing electrode is that of catalyzing the electro-reduction of oxygen. The extent of gold plating must be sufficient to permit effective reduction of the oxygen and the degree of

-4-

plating to achieve such effective reduction will be apparent to those skilled in the art. The porous sensing electrode makes contact with and has its pores wetted with potassium hydroxide electrolyte contained within the internal chamber of the sensor where a porous copper counter electrode 24 is located. The copper counter electrode is separated from the sensing electrode 22 by the insulating ring 28 which may itself be porous.

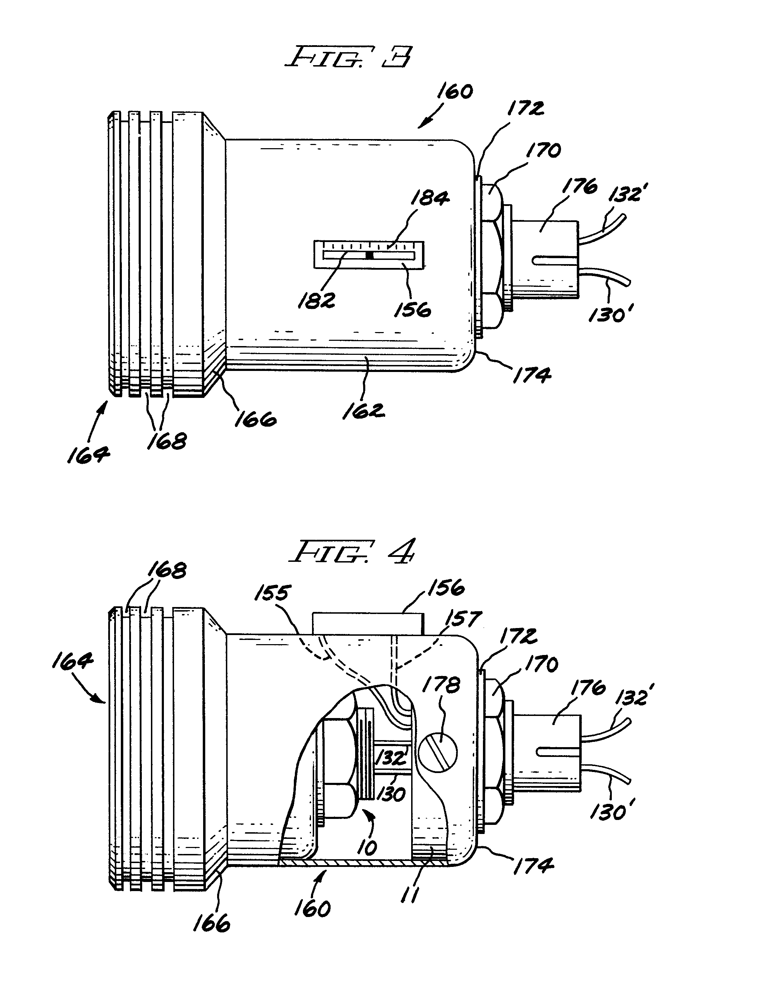

Suitable circuitry such as is illustrated in and described with reference to Figure 2 is provided between the sensing electrode 122 and the copper counter electrode 124. The elements of Figure 2 corresponding to those of Figure 1 have an assigned reference numeral which is 100 higher than the reference numerals of Figure 1. This circuitry includes connecting wires 130 and 132 and a resistance network 144 which includes thermistor 148 to compensate for temperature variations. The network 144, enclosed by dashed lines in Figure 2, is provided in parallel with a voltage meter 140 to permit a selected voltage to be developed between lines 130 and 132. The network consists of a variable resistor 146 in series with a thermistor 148, and a second variable resistor 150 in parallel with thermistor 148.

The circuitry also includes a voltmeter from which readings can be taken of the voltage drop across the resistance network 144 resulting from the current flow between the sensing electrode 122 and the porous copper counter electrode 124 to provide an indication of the partial pressure of oxygen which is in contact with the exterior surface of the polymer membrane 20 of Figure 1.

When oxygen diffuses through the polymer membrane and comes in contact with the sensing electrode 22, wetted with potassium hydroxide electrolyte, it is reduced electrochemically by electrons derived via the external wires and circuitry from the porous copper counter electrode 24 which is concurrently oxidized electrochemically. At constant temperature the rate of these reactions, and hence the associated current flow is proportional to the oxygen partial pressure in the environment being monitored. The current causes a potential drop across the resistance network 144 of Figure 2 connecting sensing electrode 22 (122) and the porous copper counter electrode 24 (124). The potential drop is measured by a voltmeter connected across the network. Wire 30 of Figure 1 is connected through wire 31 to the sensing electrode and wire 32 is connected to the porous copper electrode 24.

The following are a few of the particulars of the prior art structure to which the coulometer network 154 has been added to serve as a useful life indicator. The oxygen-diffusing membrane is a composite of fluorinated ethylene propylene polymer (FEP) and tetrafluoro ethylenepolymer (PTFE). The membrane may be formed of other polymers suitably permeable to oxygen such as polysulfone. See in this connection U.S. Patent 3,616,411 as to other alternative membranes.

The potassium hydroxide solution is a 25 weight % KOH electrolyte. The thickness of the oxygen permeable membrane is typically about 0.025 mm. The sensing electrode is a disk with a 3.3 cm diameter and a 0.14 cm thickness. The counter electrode is a copper cylinder weighing 7.6 gm. with a center bore. It has an outer diameter of 1.8 cm, an inner diameter of 0.8 cm and a length of 1.2 cm. The spacing between the sensing electrode and the counter electrode is of the order of one millimeter and is not critical.

When the sensor is exposed to air or oxygen, oxygen gas permeates through the membrane and is reduced at the sensing electrode. At the same time, the counter copper electrode is oxidized to copper oxide. The resulting current flows through an external resistor network and produces a voltage which is read on a voltmeter.

Typically, at a temperature of 75°F, and without considering the coulometer network in the circuit of Figure 2, the resistor network 144 described below has a resistance of 110 ohms. Exposure of the sensor to air at 1 atmosphere pressure produces a current of about 400 microamperes (µa) and an output voltage of 44 millivolts across the resistor network. This voltage is read with a voltmeter connected across the resistor network and with a dial calibrated to read in terms of partial pressures of oxygen. If the exposed surface of the membrane 20 of Figure 1 and the resistance values of the resistor network 144 of Figure 2 and the concentration of oxygen in air at constant temperature to which the sensor is exposed are all maintained as provided in prior art devices as specified above, the life of the prior art sensor is about 8800 hours.

The prior art oxygen sensors as described above have been used for a number of years. Sensors as described above have been supplied to NASA for a period of over eight years.

As described in a copending application, Serial No. 641,651 of the same inventors filed August 17, 1984, the sensitivity of the prior art device was not dissipated or lost if the area of the exposed sensing electrode 22 and associated membrane 20 was significantly reduced. The values of resistors 146 and 150 and thermistor 148 were also changed. In this way the same voltage outputs were obtained as in the original device but with lower currents in the new device. Surprisingly it was found that the sensitivity was retained even though the area of membrane 20 and electrode 22 is greatly reduced. This discovery and an invention resulting from the discovery is disclosed in the copending application.

The prior art oxygen sensors had relatively short overall lives of about one year. By the teaching of copending application Serial No. 641,650, filed August 17, 1984, the overall lives of such oxygen sensors was greatly extended. Sensors having overall lives in excess of one year and in fact of two or more years and of ten or twenty years were made possible by the invention disclosed in the copending application. The text of this application is incorporated herein by reference.

An astronaut or other user of prior art oxygen sensors knew that the maximum useful life of existing sensors was about one year. Any sensor installed in place in a spacecraft had to be changed or replaced within one year. By prior art practice it was necessary to keep track of the dates of installation of these sensors and to replace them within one year or less of any inspection or use. However, when the overall life of such sensors was extended and multiplied by the discovery and invention of the copending application referenced above, the situation changed very markedly. A sensor prepared according to the teaching of copending application Serial No. 641,650 could have an overall life expectancy ranging from less than one year to five or ten or fifteen years or more. Great confusion could be created in servicing of such sensors due to vastly different remaining overall life expectancies.

Use and servicing of such sensors designed for short term use of a couple of years could be greatly aided if some means were provided for determining the expended portion of an overall life expectancy as well as the residual or remaining overall life expectancy.

-8-

We have now developed such means. This is accomplished by incorporating a coulometer network into the system.

To describe and explain our novel means for determining overall residual life expectancy, reference is again made to Figure 2.

A coulometer subcircuit 154 which may be connected in series in line 132 between counter electrode 124 and resistor network 144 provides a monitoring of the history of past use of the sensor and the portion of the overall life already consumed and, reciprocally, an indication of the remaining life or life expectancy of the sensor. This subcircuit comprises a thermally compensated resistor network connected in parallel with a coulometer. The coulometer subcircuit 154 may alternatively be connected in series in line 130 between sensing electrode 122 and the resistor network 144.

Coulometer 156 is a conventional coulometer which is connected in parallel with a thermally compensated resistor network having a preferred overall resistance value of the order of one tenth to one hundredth of the resistance of the coulometer itself. This arrangement permits a known fraction of the total current to pass through the coulometer. The resistor network of subcircuit 154 is similar to resistor network 144 and includes two variable resistors 158 and 160 connected in series in line 132. A thermistor 162 is connected in parallel with variable resistor 160 as part of the network.

Coulometer 156 and the remaining elements of the network are adjusted to provide a visible indication of the degree to which the counterelectrode 124 has been oxidized and also conversely to provide a visible indication of the extent or proportion of the counterelectrode which remains unoxidized.

Chemical coulometers are preferred for use in the circuitry of the present invention. Coulometers based on transposing mercury, silver, copper, etc. across a small gap containing an aqueous ionic solution to a counter electrode, all in a capillary tube serve usefully in the combination and in the circuitry as illustrated in Figure 2. The expended life is indicated by movement of the gap along the length of the capillary as metal is transferred from one side to the other.

The purpose of the thermally compensated resistor network of subcircuit 154 is to allow for the temperature coefficient of resistance of the coulometer and to ensure that the rate and extent of change of the linear display of coulometer remains proportional to the total current at all times, and corresponds to the rate and extent of oxidation of counterelectrode 124. The rate of oxidation of counterelectrode 124 and, accordingly, the overall life of an oxygen sensor cell as described with reference to Figure 1, depends on the size of the area of the sensing electrode 122 which is exposed to the ambient atmosphere as is explained more fully in copending application Serial No. 641,650, filed August 17, 1984. Hence the values of the resistance and thermistor in coulometer network 154 will vary with the size of the sensing electrode. Introduction of the coulometer network into the circuit will also require modification in the values of the resistors and thermistor in network 144 to maintain the desired voltage output to voltmeter 140. The structure, such as is provided pursuant to the present invention may take many forms. One such form is illustrated in Figure 1. Figure 1 contains the details of the casing 10 for the oxygen sensor. The sensor, as illustrated in Figure 1, is preferably housed within an outer housing as illustrated in Figures 3 and 4. The housing 160 includes an inner cylindrical main body portion 162. The housing 160 is generally cylindrical and is adapted to contain the generally cylindrical structure of Figure 1. To mount the sensor of Figure 1 into the housing 160 of Figures 3 and 4, the casing 10 of the structure of Figure 1 is introduced into the enlarged cylindrical end 164 of the housing 160. A tapered portion 166 of housing 160 connects the inner cylindrical portion 162 and the outer enlarged cylindrical end 164. At the enlarged end 164 means, such as a number of recesses 168, may be formed to facilitate the mounting of the structure into a panel or into other supporting structure where the partial pressure of the oxygen in the atmosphere is to be sensed. At the opposite end of the housing 160, an attachment bolt 170 may be employed to hold a circuit housing 11 within the housing 160. A washer or spacer 172 is disposed between the nut 170 and the end 174 of housing 160. Other auxiliary structure 176 which may be, for example, means for holding conductors such as 130' and 132', may extend to an oxygen partial pressure display such as 140 of Figure 2 (but not shown in Figures 3 and 4). Such display is mounted in the space craft instrument panel for easy observation.

A calibrated display coulometer 156 may also be provided and attached as illustrated in the Figure 3 to the housing 160.

A site glass 182 of coulometer 156 is mounted externally of the housing 160 to permit visual inspection of the site glass 182 relative to a scale 184. Site glass 182 is a portion of the coulometer designated as 156 in Figure 2.

The coulometer used may be a conventional silver coulometer or a mercury coulometer of conventional construction having an indicating means such as a site glass.

Referring now to Figure 4, two lead wires 155 and 157 extending from coulometer 156 and corresponding to similar wires in Figure 2, are shown partially in phantom in Figure 4. The wires 155 and 157 provide electrical connection between the coulometer 156 and the circuitry which is contained within the circuit housing 11 of the oxygen sensor within housing 160.

The oxygen sensor 10 of Figure 4 is also illustrated to be electrically connected by wires 130 and 132 to circuit housing 11. The wires 130' and 132' extend from auxiliary structure 176 and can connect the sensor within housing 160 to a voltmeter such as 140 of Figure 2.

In actual use, the housing 160 may be removed from its mounting in a panel or other structure within a space craft so that the coulometer 156 can be viewed. This is all that is needed in order to make a determination as to the extent to which the entire life of the device has been consumed and the extent to which a portion of the life remains.

Adjustment of the variable resistors within the circuit housing 11 may be accomplished by turning four adjustment screws, the head 178 of one of which is shown to extend through the housing 160 so as to be available for turning adjustment from the exterior of the housing 160.

Inasmuch as the operation of conventional coulometers is generally proportional, if the observer who looks at the externally-mounted coulometer 156 on the housing 160 of a space craft oxygen sensor sees that approximately one half of the useful life has been consumed,

-12-

he may reasonably conclude that one half of the entire life of the device remains.  By marking the overall life expectancy of the sensor in years on the exterior of the housing 160 close to the externally mounted coulometer, the technician checking the oxygen sensor is informed that a certain proportion of the expected life in years has already been consumed and that a certain proportion of the life expectancy in years still remains.

# C L A I M S

1.  A portable oxygen sensor for monitoring oxygen in air having residual life indication which comprises
a sensor electrode adapted to reduce oxygen which permeates thereto through a diffusion barrier,
a counter electrode of porous copper metal,
an aqueous alkaline solution providing an electrolytic path between said electrodes,
a set of conductors and at least one resistor thermistor network providing a series electric connection between said electrodes,
a coulometer connected in parallel with a portion of said series electrical connection to indicate residual life of said sensor, and
a voltmeter connected in parallel with another portion of said series electrical connection to indicate the partial pressure of oxygen in air being monitored.

2.  The sensor of claim 1 in which the alkaline aqueous path is a solution of potassium hydroxide.

3.  The sensor of claim 1 wherein the counter electrode is of porous copper metal.

4.  The sensor of claim 1 wherein the coulometer is a mercury coulometer.

5.  The sensor of claim 1 wherein the portion of the series electrical connection in parallel with the coulometer is adjustable to conform the coulometer reading to the sensor life.

6. The sensor of claim 1 wherein the portion of the series electrical connection in parallel with the voltmeter is adjustable to conform the voltmeter reading to the partial pressure of oxygen.

7. The sensor of claim 1 in which the portion of the series electrical connection in parallel with the coulometer contains a thermistor which provides thermal compensation to said portion to ensure accurate operation of said coulometer.

8. The sensor of claim 1 in which the portion of the series electrical connection in parallel with said voltmeter contains a thermistor which provides thermal compensation to said portion to ensure accurate operation of said voltmeter.

9. The sensor of claim 1 wherein a first portion of the series electrical connection in parallel with the coulometer contains a first thermistor which provides thermal compensation to the first portion to ensure accurate operation of the coulometer and a second portion of the series electrical connection in parallel with the voltmeter contains a second thermistor which provides thermal compensation to the second portion to ensure accurate operation of the voltmeter.

---

*FIG. 1*

*FIG. 2*

# FIG. 3

# FIG. 4